# EUROPÄISCHE PATENTSCHRIFT

(11) **EP 1 303 525 B1**
(45) Veröffentlichungstag und Bekanntmachung des Hinweises auf die Patenterteilung: **08.09.2010**
(21) Anmeldenummer: 01969546.9
(22) Anmeldetag: 27.07.2001
(51) Int. Cl.: C07F 9/50

(54) **ADAMANTYLGRUPPEN ENTHALTENDE PHOSPHANLIGANDEN, DEREN HERSTELLUNG UND IHRE VERWENDUNG IN KATALYTISCHEN REAKTIONEN**
PRODUCTION OF NOVEL PHOSPHANE LIGANDS AND USE IN CATALYTICAL REACTIONS
NOUVEAUX LIGANDS DE PHOSPHANE, LEUR PRODUCTION ET LEUR UTILISATION DANS DES REACTIONS CATALYTIQUES

(30) Priorität: 27.07.2000 DE 10037961
(43) Veröffentlichungstag der Anmeldung: 23.04.2003
(73) Patentinhaber: Evonik Degussa GmbH, 45128 Essen (DE)
(72) Erfinder: BELLER, Matthias, 18119 Rostock (DE); EHRENTRAUT, Andreas, 18055 Rostock (DE); FUHRMANN, Christa, 18119 Rostock (DE); ZAPF, Alexander, 18057 Rostock (DE)
(86) Internationale Anmeldenummer: PCT/EP2001/008749
(87) Internationale Veröffentlichungsnummer: WO 2002/010178

(56) Entgegenhaltungen:
- YAMANO, TORU ET AL: "Enantioselective hydrogenation of.beta.-keto esters catalyzed by P-chira bis(dialkylphosphino)ethanes-Ru(II)" TETRAHEDRON LETT., Bd. 40, Nr. 13, 1999, Seiten 2577-2580, XP004158089
- IMAMOTO, T.; ET AL.: "P-Chiral Bis(trialkylphosphine) Ligands and Their Use in Highly Enantioselective Hydrogenation Reactions" J. AM. CHEM. SOC., Bd. 120, Nr. 7, 1998, Seiten 1635-1636, XP002186732
- LAVROVA, E. A. ET AL: "Di(1-adamantyl)phosphines" ZH. OBSHCH. KHIM. , Bd. 64, Nr. 9, 1994, Seiten 1393-1394, XP000517513
- PATSANOVSKII, I. I. ET AL: "Adamantylphosphines: effect of bulky substituent on the polar properties of sterically hindered phosphines" ZH. OBSHCH. KHIM., Bd. 64, Nr. 8, 1994, Seiten 1331-1332, XP001037966
- GOERLICH, JENS R. ET AL: "Di-1-adamantylphosphine, a highly sterically hindered phosphine. Preparation and reactions" PHOSPHORUS, SULFUR SILICON RELAT. ELEM. (1993), 81(1-4), 141-8 , Bd. 81, Nr. 1-4, 1993, Seiten 141-148, XP001038183
- YURCHENKO, R. YU. ET AL: "1-Adamantylphosphine reactions" ZH. OBSHCH. KHIM. (1986), 56(2), 482-3 , Bd. 56, Nr. 2, 1986, Seiten 425-426, XP001038184
- CHEMICAL ABSTRACTS, vol. 109, no. 9, 1987 Columbus, Ohio, US; abstract no. 67470, HACKETT, MARIFAITH, ET AL.: "Synthesis and thermolysis of dimethylbis(trialkylphosphine)platinum(II) complexes in which the phosphine ligands contain adamantyl, adamantylmethyl, and methyl groups" XP002186741 & ORGANOMETALLICS, Bd. 6, Nr. 2, 1987, Seite 403-410
- EHRENTRAUT, ANDREAS ET AL: "Palladium catalyzed reactions for fine chemical synthesis. Part 18. A new efficient palladium catalyst for Heck reactions of deactivated aryl chlorides" SYNLETT, Bd. 1, Nr. 11, 2000, Seiten 1589-1592, XP002186734
- OHASHI, A. ET AL: "A new synthetic route to unsymmetric P-chirogenic bisphosphine ligands" TETRAHEDRON LETT., Bd. 42, Nr. 6, 2001, Seiten 1099-1101, XP001026114
- STAMBULI, J.P. ET AL.: "Screening of Homogeneous Catalysts by Fluorescence Resonance Energy Transfer. Identification of Catalysts for Room-Temperature Heck Reactions" J. AM. CHEM. SOC., Bd. 123, Nr. 11, 2001, Seiten 2677-2678, XP002186735
- OHASHI, ATSUSHI ET AL: "Highly Enantioselective Hydrogenation of.alpha.-Dehydroamino Acids by Rhodium Complexes with New Unsymmetric P-Chirogenic Bisphosphine Ligands" ORG. LETT., Bd. 3, Nr. 3, 2001, Seiten 373-375, XP002186736
- STAUFFER, SHAUN R. ET AL: "Palladium-Catalyzed Arylation of Ethyl Cyanoacetate. Fluorescence Resonance Energy Transfer as a Tool for Reaction Discovery" J. AM. CHEM. SOC., Bd. 123, Nr. 19, 2001, Seiten 4641-4642, XP002186737
- ZAPF, ALEXANDER ET AL: "Palladium-catalyzed reactions for fine chemical synthesis. Part 17. A new highly efficient catalyst system for the coupling of nonactivated and deactivated aryl chlorides with arylboronic acids" ANGEW. CHEM., INT. ED. (2000), 39(22), 4153-4155 , Bd. 39, Nr. 22, 2000, Seiten 4153-4155, XP002186738

## Beschreibung

Die vorliegende Erfindung betrifft neue Phosphanliganden, deren Herstellung und ihre Verwendung in katalytischen Reaktionen, besonders zur Veredelung von Halogenaromaten.

Halogenaromaten, darunter insbesondere Chloraromaten sind vielfältig nutzbare Zwischenprodukte der chemischen Industrie, die als Vorprodukte für die Herstellung von Agrointermediaten, Pharmazeutika, Farbstoffen, Materialien, etc. dienen. Auch Vinylhalogenide sind wichtige Zwischenprodukte, die als Precursoren für Polymermonomere und die oben genannten Produkte verwendet werden.

Häufig angewandte Katalysatoren zur Funktionalisierung von Halogenaromaten oder Vinylhalogeniden zu aromatischen Olefinen bzw. Dienen (Heck-Reaktion, Stille-Reaktion), Biarylen (Suzuki-Reaktion), Alkinen (Sonogashira-Reaktion), Carbonsäurederivaten (Heck-Carbonylierung), Aminen (Buchwald-Hartwig-Reaktion) sind Palladium- und Nickelkatalysatoren. Dabei sind Palladiumkatalysatoren generell vorteilhaft, was die Breite der Anwendbarkeit von Kupplungssubstraten und teilweise die Katalysatoraktivität angeht, während Nickelkatalysatoren Vorteile im Bereich der Umsetzung von Chloraromaten und Vinylchloriden und des Metallpreises besitzen.

Palladium- und Nickelkatalysatoren, die im Rahmen der Aktivierung und weiteren Veredelung von Halogenaromaten verwendet werden, sind sowohl Palladium(II)-und/oder Nickel(II)- als auch Palladium(0)- und/oder Nickel(0)-Komplexe, obwohl es bekannt ist, daß Palladium(0)- bzw. Nickel(0)-Verbindungen die eigentlichen Katalysatoren der Reaktion sind. Insbesondere formuliert man gemäß Angaben in der Literatur koordinativ ungesättigte 14- und 16-Elektronen Palladium(0)- bzw. Nickel(0)-Komplexe, welche mit Donorliganden wie Phosphanen stabilisiert werden, als aktive Spezies.

Beim Einsatz von Iodiden als Edukten in Kupplungsreaktionen ist es möglich, auch auf Phosphanliganden zu verzichten. Allerdings sind Aryl- und Vinyliodide sehr teure Ausgangsverbindungen, die darüber hinaus stöchiometrische Mengen an Iodsalz-Abfällen produzieren. Kostengünstigere Edukte für die Heck-Reaktion wie Arylbromide oder Arylchloride benötigen den Zusatz von stabilisierenden und aktivierenden Liganden, um katalytisch produktiv wirksam zu werden.

Die für Olefinierungen, Alkinylierungen, Carbonylierungen, Arylierungen, Aminierungen und ähnliche Reaktionen beschriebenen Katalysatorsysteme weisen häufig nur mit nicht ökonomischen Ausgangsmaterialien wie lodaromaten und aktivierten Bromaromaten befriedigende katalytische Wechselzahlen ("turnover numbers" = TON) auf. Ansonsten müssen bei deaktivierten Bromaromaten und insbesondere bei Chloraromaten generell große Mengen an Katalysator - üblicherweise mehr als 1 Mol-% - zugesetzt werden, um technisch nutzbare Ausbeuten (> 90%) zu erzielen. Aufgrund der Komplexität der Reaktionsgemische ist zudem kein einfaches Katalysatorrecycling möglich, so daß auch die Rückführung des Katalysators hohe Kosten verursacht, die in der Regel einer technischen Realisierung entgegenstehen. Darüber hinaus ist es besonders bei der Herstellung von Wirkstoffen bzw. Wirkstoffvorprodukten unerwünscht, mit großen Mengen an Katalysator zu arbeiten, da ansonsten in diesem Fall Katalysatorrückstände im Produkt verbleiben. Neuere aktive Katalysatorsysteme basieren auf cyclopalladierten Phosphanen (W. A. Herrmann, C. Broßmer, K. Öfele, C.-P. Reisinger, T. Priermeier, M. Beller, H. Fischer, Angew. Chem. 1995, 107, 1989; Angew. Chem. Int. Ed. Engl. 1995, 34, 1844) oder Gemischen von sterisch anspruchsvollen Arylphosphanen (J. P. Wolfe, S. L. Buchwald, Angew. Chem. 1999, 111, 2570; Angew. Chem. Int. Ed. Engl. 1999, 38, 2413) bzw. Tri-tert. butylphosphan (A. F. Littke, G. C. Fu, Angew. Chem. 1998, 110, 3586; Angew. Chem. Int. Ed. Engl. 1998, 37, 3387) mit Palladiumsalzen oder Palladiumkomplexen.

Kostengünstige Chloraromaten sind jedoch auch mit diesen Katalysatoren generell nicht technisch befriedigend zu aktivieren, d.h. Kata lysatorproduktivitäten (TON) sind

< 10000 und Katalysatoraktivitäten (TOF) sind < 1000 h⁻¹. Somit müssen für das Erreichen von hohen Ausbeuten vergleichsweise hohe und damit sehr teure Katalysatormengen eingesetzt werden. So betragen beispielsweise die Katalysatorkosten für die Herstellung von einem Kilogramm eines organischen Zwischenprodukts mit dem Molekulargewicht 200 bei Verwendung von 1 Mol-% Palladiumkatalysator bei derzeitigen Edelmetallpreisen mehr als 100 US$, was die Notwendigkeit zur Verbesserung der Katalysatorproduktivität deutlich macht. Daher sind trotz aller Weiterentwicklungen der Katalysatoren in den letzten Jahren bis dato nur wenige industrielle Umsetzungen der Arylierung, Carbonylierung, Olefinierung etc. von Chloraromaten bekannt geworden.

Aus den genannten Gründen lag der vorliegenden Erfindung die Aufgabe zugrunde, den großen Bedarf an neuen produktiveren Katalysatorsystemen, die einfache Liganden aufweisen und die nicht die Nachteile der bekannten katalytischen Verfahren zeigen, die für die großtechnische Durchführung geeignet sind und die kostengünstige Chlor- und Bromaromaten sowie entsprechende Vinylverbindungen in hoher Ausbeute, Katalysatorproduktivität und Reinheit zu den jeweiligen Kupplungsprodukten umsetzen, zu befriedigen.

Diese Aufgabe wird erfindungsgemäß gelöst durch die Entwicklung neuer Phosphanliganden der Formel Ia,

(Adamantyl)ₙP(Alkyl)ₘ Ia

worin Adamantyl für einen in 1- oder 2-Stellung an das Phosphoratom gebundenen Adamantylrest (IIa, IIb) steht, 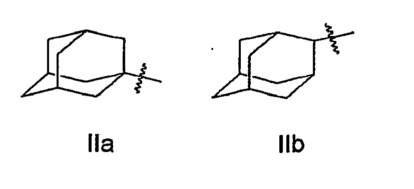 und worin Alkyl für eine C₁-bis C₁₈-Alkylgruppe
wobei sowohl die Alkylgruppe, die Alkylen'-Gruppe und der Adamantylrest unabhängig voneinander neben Wasserstoffatomen bis zu 10 Substituenten aufweisen können, die unabhängig voneinander C₁-bis C₈-Alkyl, O-Alkyl(C₁-C₈), OH, OCO-Alkyl(C₁-C₈), O-Phenyl, Phenyl, Aryl, Fluor, NO₂, SiAlkyl(C₁-C₈)₃, CN, COOH, CHO, SO₃H, NH₂, NH-Alkyl(C₁-C₈), N-Alkyl(C₁-C₈)₂, P(Alkyl(C₁-C₈))₂, P(Aryl)₂, SO₂-Alkyl(C₁-C₆), SO-Alkyl(C₁-C₆), CF₃, NHCO-Alkyl(C₁-C₄), COO-Alkyl(C₁-C₈), CONH₂, CO-Alkyl(C₁-C₈), NHCHO, NHCOO-Alkyl(C₁-C₄), CO-Phenyl, COO-Phenyl, CH=CH-CO₂-Alkyl(C₁-C₈), CH=CHCOOH, PO(Phenyl)₂, PO(Alkyl(C₁-C₄))₂, PO₃H₂, PO(OAlkyl(C₁-C₆))₂, SO₃(Alkyl(C₁-C₄)) bedeuten, wobei Aryl einen Aromaten mit 5 bis 14 Ringkohlenstoffatomen darstellt wobei ein oder mehrere Ringkohlenstoffatome durch Stickstoff-, Sauerstoff- und/oder Schwefelatome ersetzt sein können, so dass ein 1 bis 13 gliedriger Ringkohlenstoffatome enthaltender Heteroaromat vorliegt
und wobei n für eine Zahl zwischen 1 und 3 und m für eine Zahl zwischen 0 und 2 steht, wobei die Bedingung n + m = 3 erfüllt sein muß,

Insbesondere werden als Phosphanliganden erfindungsgemäß Verbindungen der Formel Ia eingesetzt, worin Adamantyl für einen in 1- oder 2-Stellung an das Phosphoratom gebundenen Adamantylrest (IIa, IIb) steht und Alkyl für eine C₁- bis C₁₂-Alkylgruppe steht.

Bevorzugt können die Alkylgruppe und der Adamantylrest unabhängig voneinander neben Wasserstoffatomen bis zu 5 Substituenten aufweisen, die unabhängig voneinander C₁- bis C₈-Alkyl, O-Alkyl(C₁-C₈), OH, OCO-Alkyl(C₁-C₈), O-Phenyl, Phenyl, Aryl, Fluor, SiAlkyl(C₁-C₈)₃, COOH, SO₃H, NH₂, NH-Alkyl(C₁-C₈), N-Alkyl₂(C₁-C₈), P(Alkyl(C₁-C₈))₂, P(Phenyl)₂, CF₃, NHCO-Alkyl(C₁-C₄), COO-Alkyl(C₁-C₈), CONH₂, CO-Alkyl(C₁-C₈), COO-Phenyl, PO(Phenyl)₂, PO(Alkyl(C₁-C₄))₂, PO₃H₂, PO(OAlkyl(C₁-C₆))₂ bedeuten, wobei Aryl für einen Aromaten mit 5 bis 14 Ringkohlenstoffatomen steht wobei auch ein oder mehrere Ringkohlenstoffatome durch Heteroatome aus der Gruppe der Stickstoff-, Sauerstoff- oder Schwefelatome ersetzt sein können, so dass ein Heteroaromat mit 4 bis 13 Ringkohlenstoffatomen vorliegt.

Heteroaromatische Reste können z. B. mindestens fünfgliedrige Ringe enthaltend 1 bis 13 Ringkohlenstoffatome sein, die bis zu 4 Stickstoffatome-und/oder bis zu 2 Sauerstoff oder Schwefelatome enthalten.
Bevorzugte heteroaromatische Arylreste enthalten ein oder zwei Stickstoff- oder ein Sauerstoff- oder ein Schwefel- oder ein Stickstoff- und ein Sauerstoff- oder Schwefelheteroatom.

Besonders bevorzugt werden als Phosphanliganden erfindungsgemäß Verbindungen der Formel Ia, worin Adamantyl für einen in 1- oder 2-Stellung an das Phosphoratom gebundenen Adamantylrest (IIa, IIb) steht und worin Alkyl für eine C₁-bis C₁₂-Alkylgruppe steht,
wobei sowohl die Alkylgruppe und der Adamantylrest unabhängig voneinander neben Wasserstoffatomen bis zu 3 Substituenten aufweisen können, die unabhängig voneinander C₁- bis C₈-Alkyl, O-Alkyl(C₁-C₈), OH, OCO-Alkyl(C₁-C₈), O-Phenyl, Phenyl, COOH, SO₃H, NH₂, P(Alkyl(C₁-C₈))₂, P(Phenyl)₂, COO-Alkyl(C₁-C₈), CONH₂, PO(Phenyl)₂ bedeuten können.

Ein weiterer Gegenstand der Erfindung ist die Herstellung der neuen Phosphanliganden. Sie werden analog zu bekannten Herstellrouten für Alkylphosphane synthetisiert. Derartige Synthesewege sind beispielsweise in Houben-Weyl, Methoden der organischen Chemie, 1963, Band XII,1 S.33 beschrieben. Generell werden die hier beschriebenen neuen Phosphanliganden durch Reaktion von Dihalogenadamantylphosphan bzw. Halogendiadamantyl-phosphan mit metallorganischen Reagenzien (beispielsweise Alkyllithium-, Alkylmagnesium-, Alkylzink- oder Alkylkupferreagenzien) hergestellt. Als Halogenadamantylphosphane sind besonders die entsprechenden Chlorverbindungen geeignet. Eine weitere Syntheseroute zur Darstellung der erfindungsgemäßen Liganden besteht in der Umsetzung von Alkaliadamantyl- oder Alkalidiadamantylphosphiden mit organischen Elektrophilen wie beispiel sweise Alkylhalogeniden, -pseudohalogeniden, Aldehyden oder Epoxiden.

Allgemein können Diadamantylalkylphosphine nach folgender Vorschrift synthetisiert werden:
Zu einer Lösung von 15 mmol Diadamantylchlorphosphin in 250 ml absolutem THF wird eine Lösung von 18 mmol R-M in THF oder Hexan getropft, wobei M Lithium oder MgHal bedeutet, wobei Hal für Chlor, Brom oder Iod steht. Die Mischung wird zwei Stunden refluxiert. Bei Raumtemperatur wird mit entgaster, wäßriger Ammoniumchloridlösung und Diethylether aufgearbeitet. Die Lösungsmittel werden abdestilliert und der Rückstand im Hochvakuum destilliert oder über Kieselgel 60 mit Hexan/Essigester-Mischungen chromatographiert.

Nach dieser Vorschrift sind z. B. die bevorzugten Liganden
Di(1-adamantyl)-i-propylphosphin,
Di(1-adamantyl)-n-butylphosphin,
Die(1-adamantyl)-n-hexylphosphin,
Di(1-adamantyl)-cyclohexylphosphin,
Die(1-adamantyl)-benzylphosphin,
Di(1-adamantyl)-pentafluorethylphosphin,
Di(3-amino-adamant-1-yl)-n-butylphosphin,
Di(3-acetyl-adamant-1-yl)-n-butylphosphin,
Di[3-(p-hydroxyphenyl)-adamant-1-yl]-methylphosphin,
Di(2-adamantyl)-i-propylphosphin,
Di(2-adamantyl)-n-butylphosphin,
Di(2-adamantyl)-t-butylphosphin,
Di(2-adamantyl)-cyclohexylphosphin,
herstellbar.

Allgemein können Adamantyldialkylphosphine nach folgender Vorschrift synthetisiert werden:
Zu einer Lösung von 35 mmol Adamantyl-M in 400 ml absolutem THF oder Hexan wird eine Lösung von 15 mmol Dialkylchlorphosphin in THF getropft, wobei M Lithium oder MgHal bedeutet, wobei Hal für Chlor oder Brom steht. Die Mischung wird vier Stunden refluxiert. Bei Raumtemperatur wird mit entgaster, wäßriger Ammoniumchloridlösung und Diethylether aufgearbeitet. Die Lösungsmittel werden abdestilliert und der Rückstand im Hochvakuum destilliert oder über Kieselgel 60 mit Hexan/Essigester-Mischungen chromatographiert.

Nach dieser Vorschrift können z. B. die bevorzugten Liganden,
(1-Adamantyl)-di-cyclohexylphosphin,
(2-Adamantyl)-di-n-butylphosphin,
hergestellt werden.

Die neuen Phosphanliganden werden erfindungsgemäß in Kombination mit Übergangsmetallkomplexen oder Übergangsmetallsalzen der VIII. Nebengruppe des Periodensystems der Elemente wie beispielsweise Palladium, Nickel, Platin, Rhodium, Iridium, Ruthenium, Cobalt als Katalysatoren eingesetzt. Dabei können die erfindungsgemäßen Liganden in der Regel in situ zu entsprechenden Übergangsmetallprecusorverbindungen gegeben werden und so für katalytische Anwendungen verwendet werden.

Als Übergangsmetallverbindungen werden bevorzugt Palladium- oder NickelVerbindungen und besonders bevorzugt Palladium-Verbindungen eingesetzt.

Mitunter kann es vorteilhaft sein, daß definierte Mono-, Di-, Tri- oder Tetraphosphankomplexe der genannten Übergangsmetalle zunächst hergestellt werden, die dann für Katalysereaktionen verwendet werden.

Bevorzugt ist die Verwendung von Palladium- und Nickelkatalysatoren, die die erfindungsgemäßen Phosphane enthalten.

Besonders bevorzugt ist die Verwendung von Palladiumkatalysatoren mit den erfindungsgemäßen Liganden. Die erfindungsgemäßen Liganden werden in der Regel in situ zu Palladium(II)salzen bzw. Palladium(II)- bzw. Palladium(0)komplexen zugegeben. Es kann jedoch vorteilhaft sein, daß Palladium(0)- bzw. Palladium(II)-Phosphan-Komplexe der erfindungsgemäßen Phosphane direkt hergestellt und dann für Katalyseanwendungen eingesetzt werden. Mitunter ist dadurch die anfängliche katalytische Aktivität erhöht.

Als Palladiumkomponente können mit den erfindungsgemäßen Liganden beispielsweise eingesetzt werden: Palladium(II)acetat, Palladium(II)chlorid, Palladium(II)bromid, Lithiumtetrachloropalladat(II), Palladium(II)acetylacetonat, Palladium(0)-dibenzylidenaceton-Komplexe, Palladium(0)tetrakis(triphenylphosphan), Palladium(0)bis(tri-o-tolylphosphan), Palladium(II)propionat, Palladium(II)-bis(triphenylphosphan)dichlorid, Palladium(0)diallylether-Komplexe, Palladium(II)nitrat, Palladium(II)chlorid-bis(acetonitril), Palladium(II)chlorid-bis(benzonitril) und weitere Palladium(0)- und Palladium(II)-Komplexe.

Im allgemeinen wird der Phosphanligand bei katalytischen Anwendungen im Überschuß zum Übergangsmetall eingesetzt. Das Verhältnis Übergangsmetall zu Ligand beträgt vorzugsweise von 1 : 1 bis 1 : 1000. Besonders bevorzugt werden Verhältnisse von Übergangsmetall zu Ligand von 1 : 1 bis 1 : 100. Das genaue zu verwendende Übergangsmetall/Ligand-Verhältnis hängt von der konkreten Anwendung, aber auch von der eingesetzten Katalysatormenge ab. So ist es generell üblich, bei sehr niedrigen Übergangsmetallkonzentrationen (< 0.01 Mol-%) niedrige Übergangsmetall/Ligand-Verhältnisse zu verwenden als bei Übergangsmetallkonzentrationen zwischen 0.5 und 0.01 Mol-% Übergangsmetall.

Die neuen Phosphanliganden sind thermisch sehr stabil. So können die erfindungsgemäßen Katalysatoren bei Reaktionstemperaturen bis über 250°C verwendet werden. Vorzugsweise werden die Katalysatoren bei Temperaturen von 20 bis 200 °C eingesetzt; in vielen Fällen hat es sich bewährt, bei Temperaturen von 30 bis 180 °C, bevorzugt 40 bis 160°C, zu arbeiten. Die Liganden können ohne Verlust an Aktivität auch bei Druckreaktionen verwendet werden, wobei üblicherweise nur bis zu einem Druck von 100 bar gearbeitet wird, vorzugsweise jedoch im Bereich des Normaldrucks bis zu 60 bar.

Die erfindungsgemäß hergestellten Phosphanliganden haben sich insbesondere als Ligandenkomponente zur katalytischen Herstellung von arylierten Olefinen (Heck-Reaktionen), Biarylen (Suzuki-Reaktionen), und Aminen aus Arylhalogeniden oder Vinylhalogeniden bewährt. Für den Fachmann ist es jedoch naheliegend, daß auch andere übergangsmetallkatalysierte Reaktionen wie die Metathese oder Hydrierungen von Doppelbindungen bzw. Carbonylverbindungen, insbesondere jedoch palladium- und nickelkatalysierte Carbonylierungen von Arylhalogeniden, Alkinylierungen mit Alkinen (Sonogashira-Kupplungen), Kreuzkupplungen mit metallorganischen Reagenzien (Zinkreagenzien, Zinnreagenzien etc.) mit den neuen Katalysatorsystemen katalysiert werden können.

Für einige Katalyseanwendungen wie beispielsweise Carbonylierungen kann der Einsatz von chelatisierenden Phosphanliganden von Vorteil sein. Dabei sind insbesondere chelatisierende Phosphanliganden mit einer aliphatischen Kohlenstoffbrücke von C₂ bis C₆ oder mit aromatischer Brücke (1,2-Phenylen, Ferrocenyl, Binaphthyl) von Bedeutung.

Ein besonderer Vorteil der erfindungsgemäßen Liganden ist die hohe Aktivität, die die Liganden bei der Aktivierung von kostengünstigen jedoch inerten Chloraromaten induzieren. Wie in den Versuchsbeispielen gezeigt, übertreffen Palladiumkatalysatoren mit den neuen Adamantylphosphanen die bis dato besten Katalysatorsysteme von Buchwald (J. P. Wolfe, S. L. Buchwald, Angew. Chem. 1999, 111, 2570; Angew. Chem. Int. Ed. Engl. 1999, 38, 2413) und Fu (A. F. Littke, G. C. Fu, Angew. Chem. 1998, 110, 3586; Angew. Chem. Int. Ed. Engl. 1998, 37, 3387) signifikant. Somit können mit den erfindungsgemäßen Katalysatorsystemen auch mit Chloraromaten als Substrate Turnover-Werte in der Größenordnung von > 10 000 und für Bromaromaten als Ausgangsmaterialien TONs > 500 000 realisiert werden. Damit sind die beschriebenen Katalysator- und Ligandensysteme für großtechnische Zwecke nutzbar.

Besonders überraschend sind die Eigenschaften der Adamantylphosphane. Obwohl Adamantylreste in der organischen Chemie seit langem bekannt sind, wurde Phosphanliganden mit Adamantylgruppen keine Bedeutung zugebilligt. Daher wurden Alkyladamantylphosphane bis dato auch nicht für katalytische Anwendungen beschrieben. Überraschend war, daß Adamantylliganden alle anderen bekannten Phosphanliganden in bestimmten Katalyseanwendungen signifikant übertreffen. Während z. B. mit den besten bisher bekannten Palladiumkatalysatoren bei Verwendung geringer Katalysatormengen (0.005 Mol-%) Produktausbeuten bei der Kupplung von 4-Chlortoluol mit Arylboronsäure von 16 bis 46% erzielt werden, wurden Ausbeuten > 90 % mit den erfindungsgemäßen Liganden erhalten.

Die erfindungsgemäß hergestellten Phosphane können für die Herstellung von Arylolefinen, Dienen, Diarylen, Benzoesäurederivaten, Acrylsäured erivaten, Arylalkanen, Alkinen, Aminen eingesetzt werden. Die so hergestellten Verbindungen können unter anderem eingesetzt werden als UV-Absorber, als Zwischenprodukte für Pharmazeutika und Agrochemikalien, als Ligandvorstufen für Metallocenkatalysatoren, als Riechstoffe, Wirkstoffe und Bausteine für Polymere.

### Ausführungsbeispiele:

Die nachstehenden Beispiele dienen zur Erläuterung der Erfindung, ohne sie darauf zu beschränken.

Allgemeines: Die Herstellung der Adamantylphosphinliganden erfolgt unter Schutzgas (Argon).

### Allgemeine Vorschrift zur Phosphinsynthese:

Eine Mischung von 100 g (0.73 mol) Adamantan, 105 g (0.79 mol) Aluminium(III)chlorid und 300 ml Phosphor(III)chlorid wurde 5 h refluxiert. Der Überschuß an Phosphor(III)chlorid wurde abdestilliert, bis eine rot-braune, viskose Substanz zurückblieb. Nachdem diese in 1 l Chloroform suspendiert wurde, wurde mit 1 I Eiswasser hydrolysiert. Die organische Phase wurde über Natriumsulfat getrocknet und im Vakuum (0.1 mbar) zur Trockene eingeengt. Ausbeute: 130 g (0.37 mol, 93 %) Di(1-adamantyl)phosphinylchlorid (Schmelzpunkt: 195 °C).

40 g Diadamantylphosphinylchlorid (0.11 mol) wurden in 600 ml absolutiertem Tetrahydrofuran vorgelegt, mit Eiswasser / Natriumchlorid-Kältemischung auf -14°C gekühlt und 10 g (0.26 mol) Lithiumaluminiumhydrid über 60 min sukzessive zugegeben. Die Mischung wurde dann bei Raumtemperatur 16 h gerührt und bei -14°C mit 200 ml einer 1n HCl-Lösung hydrolysiert. Die organische Phase wurde über Natriumsulfat getrocknet und im Vakuum (0.1 mbar) zur Trockene eingeengt.
Ausbeute: 30 g (0.10 mol, 94 %) Di(1-adamantyl)phosphin.
³¹P- NMR (162.0 MHz, CDCl₃): δ = 18.2

Bei -14 °C wurde zu einer Lösung von 23 g (76 mmol) Di(1-adamantyl)phosphin und 14.5 g (9.5 mmol) 1,8-Diazabicylo[5.4.0]undec-7-en (DBU) in 600 ml Toluol 60 g einer 20 %igen Lösung von Phosgen in absolutem Toluol zugetropft und nach Erwärmen auf Raumtemperatur noch 16 h gerührt. Die Mischung wurde filtriert und das Lösungsmittel im Vakuum abdestilliert.
Ausbeute: 23 g (68 mmol, 90 %) Diadamantylchlorphosphin.
³¹P- NMR (162.0 MHz, CDCl₃): δ = 138.4

### Beispiel 1

### Di(1-adamantyl)-n-butylphosphin (nBuPAd₂) (Variante 1):

Zu 5.0 g (15 mmol) Diadamantylchlorphosphin wurden in 250 ml absolutiertem Tetrahydrofuran wurden 11 ml einer 1.6 M Lösung von *n*-Butyllithium in Hexan (18 mmol) zugetropft. Die Lösung wurde 1 h refluxiert. Nach Entfernen des Lösungsmittels im Vakuum wurde der Rückstand im Vakuum destilliert.
Es wurden 2.6 g (7.3 mmol, 49 %) Diadamantyl-*n*-butylphosphin erhalten.

### Di(1-adamantyl)-n-butylphosphin (nBuP(1-Ad)₂) (Variante 2):

4.6 g (15 mmol) Di(1-adamantyl)phosphin wurden in 50 ml Di-*n*-butylether vorgelegt und 20 ml einer 2.5M Lösung von *n-*BuLi in Toluol (50 mmol) zugegeben. Die Mischung wurde 1 h unter Rückfluß gekocht, abgekühlt und 4.1 g (30 mmol) 1-Butylbromid zugetropft. Die Mischung wurde 30 min unter Rückfluß gekocht, abgekühlt, mit gesättigter Ammoniumchloridlösung gewaschen (3x), die organische Phase abgetrennt, über Natriumsulfat getrocknet und das Lösungsmittel unter vermindertem Druck abdestilliert.
Ausbeute: 4.6 g (13 mmol, 85%) Di(1-adamantyl)-*n*-butylphosphin. Das Produkt kann aus Di-*n*-butylether umkristallisiert werden (Smp. 102°C).
³¹P {¹H} - NMR (162.0 MHz, C₆D₆, 297 K): δ = 24.6
MS (E.I., 70 eV): *m*/*z*: 358 (M⁺, 12%); 135 (Ad⁺, 100%)
MS (C.I., Isobuten): *m*/*z*: 359 (M⁺+ H, 100%)

### Di(1-adamantyl)-n-butylphosphin (nBuP(1-Ad)₂) (Variante 3):

1.5 g (4.5 mmol) Di(1-adamantyl)chlorphosphin wurden in 40 ml absolutem THF vorgelegt und unter Rühren wurde mit einer Spritze 5 ml einer 1.6M *n*BuLi - Lsg. in Hexan (8 mmol) zugegeben. Die Mischung wurde 2h unter Rückfluß gekocht, das Lösungsmittel unter vermindertem Druck abdestilliert und der Rückstand im Kugelrohr destilliert.
Ausbeute: 0.77 g (2.1 mmol, 48%) Di(1-adamantyl)-*n*-butylphospin.

### Di(1-adamantyl)-n-butylphosphin (nBuP(1-Ad)₂) (Variante 4):

4.6 g (15 mmol) Di(1-adamantyl)phosphin wurden in 50 ml Di*-n-*butylether vorgelegt und 20 ml einer 2.5M Lösung von *n*-BuLi in Toluol (50 mmol) zugegeben. Die Mischung wurde 1 h unter Rückfluß gekocht, abgekühlt und 2.8 g (30 mmol) 1-Butylchlorid wurde zugetropft. Die Mischung wurde 30 min unter Rückfluß gekocht, abgekühlt, mit gesättigter Ammoniumchloridlösung gewaschen (3x), die organische Phase abgetrennt, über Natriumsulfat getrocknet und das Lösungsmittel unter vermindertem Druck abdestilliert. Das Produkt wurde mittels Kugelrohr-Destillation im Feinvakuum gereinigt.
Ausbeute: 4.6 g (13 mmol, 85%) Di(1-adamantyl)*-n-*butylphosphin.

### Beispiel 2 (Verbindungen zählen nicht zu den neuen erfindungsgemässen Phosphanliganden.)

### Di(1-adamantyl)-methylphosphin (MeP(1-Ad)₂) (Variante 1):

Zu 5.0 g (15 mmol) Diadamantylchlorphosphin in 250 ml absolutiertem Tetrahydrofuran wurden 11 ml einer 1.6 M Lösung von Methyllithium in Hexan (18 mmol) zugetropft. Die Lösung wurde 1 h refluxiert. Nach Abdestillieren des Lösungsmittels im Vakuum wurde der Rückstand im Vakuum destilliert.
Es wurden 2.3 g (7.3 mmol, 49 %) Diadamantylmethylphosphin erhalten.

### Di(1-adamantyl)-methylphosphin (MeP(1-Ad)₂) (Variante 2):

2.0 g (6.0 mmol) Di(1-adamantyl)chlorphosphin wurden in 50 ml absolutem THF vorgelegt und unter Rühren wurden mit einer Spritze 5 ml einer 1.6M MeLi - Lsg. in Diethylether (8 mmol) zugegeben. Die Mischung wurde 2h unter Rückfluß gekocht, das Lösungsmittel unter vermindertem Druck abdestilliert und der Rückstand im Kugelrohr destilliert.
Ausbeute: 0.85 g (2.7 mmol, 45%) Di(1-adamantyl)-methylphospin (Smp. 143°C). Elementaranalyse gef. (ber.): C: 79.52% (79.70%); H: 10.60% (10.51%); P: 9.78% (9.79%).
³¹P {¹H} - NMR (162.0 MHz, C₆D₆, 297 K): δ = 7.8
MS (E.I., 70 eV): m/z: 316 (M⁺ , 36%); 135 (Ad⁺, 100%)

### Beipiel 3

### Di(1-adamantyl)-n-hexylphosphin (HexP(1-Ad)₂) (Variante 1):

In 150 ml absolutiertem Tetrahydrofuran wurden 0.45 g Magnesiumspäne (18 mmol) vorgelegt und unter Rühren mit 3.0 g 1-Bromhexan (18 mmol) versetzt, so daß der Ether sich erwärmte. Nach dem Abkühlen der Mischung auf Raumtemperatur wurde eine Lösung von 5.0 g Diadamantylchlorphosphin (15 mmol) in 100 ml absolutiertem Tetrahydrofuran zugetropft und 1 h refluxiert. Nach Abdestillieren des Lösungsmittels im Vakuum wurde der Rückstand im Hochvakuum (0.01 mbar) destilliert.
Ausbeute: 2.0 g (5.2 mmol, 35 %) Diadamantyl*-n-*hexylphosphin.

### Di(1-adamantyl)-n-hexylphosphin (HexP(1-Ad)₂) (Variante 2):

5.5 g (18 mmol) Di(1-adamantyl)phosphin wurden in 60 ml Di*-n-*butylether vorgelegt und 20 ml einer 2.5M Lösung von *n-*BuLi (50 mmol) in Toluol zugegeben. Die Mischung wurde 45 min unter Rückfluß gekocht, abgekühlt und 3.0 g (18 mmol) 1-Bromhexan zugetropft. Die Mischung wurde 30 min unter Rückfluß gekocht, abgekühlt, mit gesättigter Ammoniumchloridlösung gewaschen (3x), die organische Phase abgetrennt, über Natriumsulfat getrocknet und das Lösungsmittel unter vermindertem Druck abdestilliert.
Ausbeute: 4.9 g (13 mmol, 70%) Di(1-adamantyl)*-n-*hexylphosphin. Das Produkt kann aus Di*-n-*butylether umkristallisiert werden.
³¹P {¹H} - NMR (162.0 MHz, C₆D₆, 297 K): δ = 24.6
MS: 386.31062 (Calc. 386.31024 for C₂₆H₄₃P)

### Beipiel 4 (Verbindungen zählen nicht zu den neuen erfindungsgemässen Phosphanliganden.)

### Bis-(diadamantylphosphino)butan (Butylen(PAd₂)₂):

In 150 ml absolutiertem Tetrahydrofuran wurden 0.45 g Magnesiumspäne (18 mmol) vorgelegt und unter Rühren mit 2.0 g 1,4-Dibrombutan (9.3 mmol) versetzt, so daß der Ether sich erwärmte. Nach dem Abkühlen der Mischung auf Raumtemperatur wurde eine Lösung von 5.0 g Diadamantylchlorophosphin (15 mmol) in 100 ml absolutiertem Tetrahydrofuran zugetropft und 1h refluxiert. Nach Abdestillieren des Lösungsmittels im Vakuum wurde der Rückstand im Hochvakuum (0.01 mbar) destilliert.
Ausbeute: 1.0 g (1.5 mmol, 10 %) Bis-(diadamantylphosphino)butan.

### Beispiel 5

### Di(1-adamantyl)-3-dimethylaminopropylphosphin:

5.1 g (17 mmol) Di(1-adamantyl)phosphin wurden in 50 ml Di*-n-*butylether vorgelegt und 20 ml einer 2.5M Lösung von *n-*BuLi (50 mmol) in Toluol zugegeben. Die Mischung wurde 1 h unter Rückfluß gekocht, abgekühlt und unter Kühlung im Eisbad 5.0 g (31 mmol) 3-Dimethylaminopropylchlorid-Hydrochlorid zugegeben. Die Mischung wurde 30 min unter Rückfluß gekocht, abgekühlt, mit gesättigter Ammoniumchloridlösung gewaschen (3x), die organische Phase abgetrennt, über Natriumsulfat getrocknet und das Lösungsmittel unter vermindertem Druck abdestilliert. Ausbeute: 4.6 g (12 mmol, 70%) Di(1-adamantyl)-3-dimethylaminopropylphosphin. Das Produkt kann aus Di*-n-*butylether umkristallisiert werden (Smp. 138°C).
Elementaranalyse gef. (ber.): C: 77.46% (77.47%); H: 11.09% (10.92%); N: 3.47% (3.61 %); P: 7.78% (7.99%).
³¹P {¹H} - NMR (162.0 MHz, C₆D₆, 297 K): δ = 24.5
MS: 387.30528 (Calc. 387.30548 for C₂₅H₄₂NP)

### Beispiel 6

### Di(1-adamantyl)-benzylphosphin:

4.0 g (13 mmol) Di(1-adamantyl)phosphin wurden in 50 ml Di*-n-*butylether vorgelegt und 18 ml einer 2.5M Lösung von *n-*BuLi (45 mmol) in Toluol zugegeben. Die Mischung wurde 30 min unter Rückfluß gekocht, abgekühlt und 3.2 g (19 mmol) Benzylbromid zugetropft. Die Mischung wurde 30 min unter Rückfluß gekocht, abgekühlt, mit gesättigter Ammoniumchloridlösung gewaschen (3x), die organische Phase abgetrennt, über Natriumsulfat getrocknet und das Lösungsmittel unter vermindertem Druck abdestilliert. Ausbeute: 4.6 g (12 mmol, 90%) Di(1-adamantyl)benzylphosphin. Das Produkt wird aus Di*-n-*butylether umkristallisiert (Smp. 182°C).
³¹P{¹H} - NMR (162.0 MHz, C₆D₆, 297 K): δ = 29.8
MS: 392.26420 (Calc. 392.26328 for C₂₇H₃₇P)

### Beispiel 7 bis 20

### Allgemeine Arbeitsvorschrift zur Heck-Reaktion:

In einem Druckrohr (erhältlich z.B. bei Fa. Aldrich) wurden unter einer ArgonAtmosphäre 5 mmol Arylhalogenid, 6 mmol Olefin, 6 mmol Base, eine geeignete Menge an Ligand und Palladium(0)-dba-Komplex sowie 500 mg Diethylenglycoldi-*n-*butylether (als interner Standard für die GC-Analytik) zu 5 ml absolutiertem Dioxan gegeben. Das Rohr wurde verschlossen und in ein 120 °C heißes Siliconölbad gehängt. Nach 24 h ließ man es auf Raumtemperatur abkühlen. Die Feststoffe wurden in 5 ml Methylenchlorid und 5 ml 2 n Salzsäure gelöst. Die organische Phase wurde gaschromatographisch analysiert. Die Produkte wurden durch Destillation, Kristallisation aus Methanol/Aceton-Mischungen oder säulenchromatographisch (Kieselgel, Hexan/Ethylacetat-Mischungen) isoliert.

| Tabelle 1: Heck-Reaktion von p-Chlortoluol und Styrol; *n*BuPAd₂ als Ligand | | | | | | | |
|---|---|---|---|---|---|---|---|
| Nr. | Base | Temp. (°C) | Kat.konz. (Mol-%) | L:Pd | Umsatz (%) | Ausbeute (%) | TON |
| 7 | K₃PO₄ | 100 | 1.0 | 1:1 | 42 | 38 | 38 |
| 8 | K₃PO₄ | 100 | 1.0 | 2:1 | 39 | 25 | 25 |
| 9 | K₃PO₄ | 120 | 0.1 | 2:1 | 27 | 20 | 200 |
| 10 | K₃PO₄ | 120 | 1.0 | 2:1 | 98 | 98 | 98 |
| 11 | K₃PO₄ | 120 | 0.1 | 4:1 | 25 | 11 | 110 |
| 12 | K₂CO₃ | 120 | 1.0 | 2:1 | 78 | 68 | 68 |
| 13 | K₃PO₄ | 140 | 0.1 | 4:1 | 88 | 81 | 810 |

| Tabelle 2: Heck-Reaktion von Chlorbenzol und Styrol, bei 120 °C, L:Pd = 2:1 | | | | | |
|---|---|---|---|---|---|
| Nr. | Base | Kat.konz. (Mol-%) | Umsatz. (%) | Ausbeute (%) | TON |
| 14 | K₂CO₃ | 1.0 | 71 | 63 | 63 |
| 15 | K₃PO₄ | 2.0 | 46 | 33 | 17 |

| Tabelle 3: Heck-Reaktion mit Acrylsäure-2-ethylhexylester, bei 120 °C, Base: K₃PO₄, 2.0 mol-% Pd(dba)₂, L:Pd = 2:1 | | | | | |
|---|---|---|---|---|---|
| Nr. | Arylchlorid | Ligand | Umsatz (%) | Ausbeute (%) | TON |
| 16 | 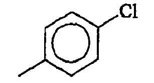 | nBuPAd₂ | 66 | 63 | 32 |
| 17 | 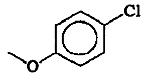 | nBuPAd₂ | 94 | 82 | 41 |
| 18 | 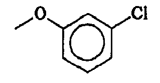 | nBuPAd₂ | 51 | 34 | 17 |
| 19 | 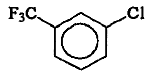 | nBuPAd₂ | 38 | 12 | 6 |
| 20 | 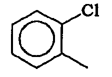 | nBuPAd₂ | 48 | 44 | 22 |

### Beispiele 21 bis 40

### Allgemeine Arbeitsvorschrift zur Suzuki-Reaktion:

In einem Druckrohr (erhältlich z.B. bei Fa. Aldrich) wurden unter einer ArgonAtmosphäre 3 mmol Arylhalogenid , 4.5 mmol Phenylboronsäure, 6 mmol Base, eine geeignete Menge an Ligand und Palladium(II)acetat (P/Pd=2:1) sowie 100 mg Hexadecan (als interner Standard für die GC-Analytik) in 6 ml absolutiertem Toluol gelöst. Das Rohr wurde verschlossen und in ein 100 °C heißes Siliconölbad gehängt. Nach 20 h ließ man es auf Raumtemperatur abkühlen. Die Feststoffe wurden in 10 ml Methylenchlorid und 10 ml verdünnter Natronlauge gelöst. Die organische Phase wurde gaschromatographisch analysiert. Die Produkte wurden durch Kristallisation aus Methanol/Aceton-Mischungen oder säulenchromatographisch (Kieselgel, Hexan/Ethylacetat-Mischungen) isoliert.

| Tabelle 4: Einfluß des Liganden auf die Kupplung von 4-Chlortoluol und Phenylboronsäure. | | | | |
|---|---|---|---|---|
| Nr. | PR₃ | Pd(OAc)₂ (Mol-%) | Ausbeute (%) | TON |
| 21 | PPh₃ | 0.1 | 5 | 50 |
| 22 | PhPCy₂ | 0.1 | 23 | 230 |
| 23^{[a]} | (o-tol)PCy₂ | 0.1 | 49 | 490 |
| 24^{[a]} | (o-anisyl)PCy₂ | 0.1 | 42 | 420 |
| 25 | (o-biph)PCy₂ | 0.01 | 47 | 4 700 |
| 26 | PCy₃ | 0.1 | 23 | 230 |
| 27 | P^{t}Bu₃ | 0.01 | 92 | 9 200 |
| 28 | P^{t}Bu₃ | 0.005 | 41 | 8200 |
| 29 | BuPAd₂ | 0.01 | 94 | 9 400 |
| 30 | BuPAd₂ | 0.005 | 87 | 17 400 |

| | | | | |
|---|---|---|---|---|
| [a] P/Pd = 4:1. | | | | |

| Tabelle 5: Suzuki-Kupplung verschiedener Arylchloride (R-C₆H₄-Cl) mit Phenylboronsäure in Gegenwart von 0.005 Mol-% Pd(OAc)₂/2 BuPAd₂. | | | |
|---|---|---|---|
| Nr. | R | Ausbeute (%) | TON |
| 31 | 4-Me | 87 | 17 400 |
| 32^{[a]} | 4-Me | 74 | 14 800 |
| 33 | 2-Me | 85 | 17 000 |
| 34 | 2,6-Me₂ | 68 | 13 600 |
| 35 | H | 80 | 16 000 |
| 36 | 2-F | 96 | 19 200 |
| 37 | 4-MeO | 64 | 12 800 |
| 38 | 3-MeO | 58 | 11 600 |
| 39 | 2-CN | 100 | 20000 |
| 40 | "3-N"^{[b]} | 99 | 19 800 |

| | | | |
|---|---|---|---|
| [a] 4 statt 20 h; [b] 3-Chlorpyridin. | | | |

### Beispiele 41 bis 54

### Allgemeine Arbeitsvorschrift zur katalytischen Aminierung:

In einem Druckrohr (erhältlich z.B. bei Fa. Aldrich) wurden unter einer ArgonAtmosphäre 5 mmol Arylhalogenid , 6 mmol Amin, 6 mmol Natrium-fert-butylat, eine geeignete Menge an Ligand und Palladium(0)-Dibenzylidenaceton - Komplex zu 5 ml absolutiertem Toluol gegeben. Das Rohr wurde verschlossen und in ein 120 °C heißes Siliconölbad gehängt. Nach 20 h ließ man es auf Raumtemperatur abkühlen. Die Feststoffe wurden in 5 ml CH₂Cl₂ und 5 ml 2 n Salzsäure gelöst und 500 mg Diethylenglykoldi*-n-*butylether als interner GC-Standard hinzugegeben. Die organische Phase wurde gaschromatographisch analysiert. Die Produkte wurden durch Destillation, Kristallisation aus Methanol/Aceton-Mischungen oder säulenchromatographisch (Kieselgel, Hexan/Ethylacetat-Mischungen) isoliert.

| Tabelle 6: Katalytische Aminierung von Arylhalogeniden; 0,5 mol% Pd(dba)₂, nBuPAd₂ | | | | |
|---|---|---|---|---|
| Nr. | Arylchlorid | Amin | Produkt | Ausbeute [%] |
| 41 | 2-Chlor-m-xylol | 2,6-Dimethylanilin | Bis(2,6-dimethylphenyl)amin | 84 |
| 42 | 2-Chlor-m-xylol | 2,6-Diisopropylanilin | 2,6-Dimethylphenyl-2',6'-diisopropylanilin | 70 |
| 43 | 2-Chlorfluorbenzol | 2,6-Diisopropylanilin | 2-Fluorphenyl-2',6'-diisopropylanilin | 70 |
| 44 | 2-Chlor-m-xylol | 1-Adamantylamin | N-(1-Adamantyl)-2,6-dimethylanilin | 84 |
| 45 | 2-Chlor-m-xylol | *tert*.-Butylamin | N-(*tert*.-Butyl)-2,6-dimethylanilin | 93 |
| 46 | Chlorbenzol | Diethylamin | N-N-Diethylanilin | 44 |
| 47 | Chlorbenzol | Di*-n-*butylamin | N,N-Di*-n-*butylanilin | 72 |
| 48 | 3-Chlortoluol | Diethylamin | N,N-Diethyl-*m*-toluidin | 49 |
| 49 | 3-Chloranisol | Diethylamin | N,N-Diethyl-*m*-methoxyanilin | 58 |
| 50 | 4-Chlortoluol | Diethylamin | N,N-Diethyl-*p*-toluidin | 40 |
| 51 | Chlorbenzol | Piperidin | N-Phenylpiperidin | 76 |
| 52 | Chlorbenzol | Morpholin | N-Phenylmorpholin | 87 |
| 53 | o-Chloranisol | 2,6-Dimethylanilin | 2-Methoxyphenyl-2,6-dimethylanilin | 100 |
| 54 | o-Chloranisol | 2,6-Diisopropylanilin | 2-Methoxyphenyl-2,6-diisopropylanilin | 88 |

### Beispiele 55 bis 59

### Katalytische Keton-α-Arylierung:

In einem Druckrohr (erhältlich z.B. bei Fa. Aldrich) wurden unter einer ArgonAtmosphäre 5 mmol Arylhalogenid , 6 mmol Keton, 6 mmol Natrium-*tert*-butylat, eine geeignete Menge an Ligand und Palladium(II)-acetat zu 5 ml absolutiertem Toluol gegeben. Das Rohr wurde verschlossen und in ein 80 °C heißes Siliconölbad gehängt. Nach 20 h ließ man es auf Raumtemperatur abkühlen. Die Feststoffe wurden in 5 ml CH₂Cl₂ und 5 ml 2 *n* Salzsäure gelöst und 500 mg Diethylenglykoldi-*n-*butylether als interner GC-Standard hinzugegeben. Die organische Phase wurde gaschromatographisch analysiert. Die Produkte wurden durch Destillation, Kristallisation aus Methanol/Aceton-Mischungen oder säulenchromatographisch (Kieselgel, Hexan/Ethylacetat-Mischungen) isoliert.

| Tabelle 7: Katalytische Keton-α-Arylierung; 1mol% PdOAc₂; 2mol% nBuPAd₂ | | | | | | | | |
|---|---|---|---|---|---|---|---|---|
| Nr. | Aryl-X | T (°C) | Keton | Umsatz (%) | Produkt 1 (monoaryliert) | Ausbeute^{a}) (%) | Produkt 2 (bisaryliert) | Ausbeute^{a}) (%) |
| 55 | 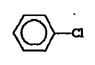 | 120 | 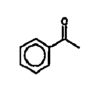 | 100 | 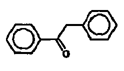 | 70 | 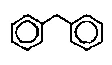 | 28 |
| | Chlorbenzol | | Acetophenon | | Desoxybenzoin | | Diphenylmethan | |
| 56 | 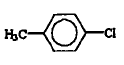 | 80 | 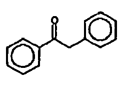 | 66 | ./. | | 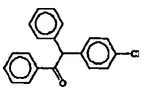 | 65 |
| | p-Chlortoluol | | Desoxybenzoin | | | | 1,2-Diphenyl-2-p-tolylethanone | |
| 57 | 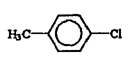 | 80 | 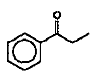 | 99 | 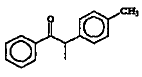 | 97 | ./. | |
| | p-Chlortoluol | | Propiophenon | | 1-Phenyl-2-p-tolylpropan-1-one | | | |
| 58 | 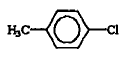 | 80 | 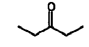 | 100 | 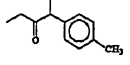 | 54 | nicht isoliert | k.A. |
| | p-Chlortoluol | | 3-Pentanon | | 2-p-Tolylpentan-3-one | | | |
| 59 | 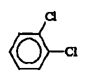 | 80 | 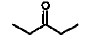 | 100 | 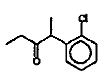 | 58 | nicht isoliert | k. A. |
| | 1,2-Dichlorbenzol | | 3-Pentanon | | 2-(2'-Chlorophenyl)-pentan-3-one | | | |

### Beispiele 60 bis 79

### Weitere Katalysebeispiele zur Keton -α-Arylierung:

In einem Druckrohr (erhältlich z.B. bei Fa. Aldrich) wurden unter einer ArgonAtmosphäre 5 mmol Arylhalogenid , 6 mmol Keton, 6 mmol Trikaliumphosphat, eine geeignete Menge an Ligand und Palladium(II)-acetat zu 5 ml absolutiertem Dioxan gegeben. Das Rohr wurde verschlossen und in ein 100°C heißes Siliconölbad gehängt. Nach 20 h ließ man es auf Raumtemperatur abkühlen. Die Feststoffe wurden in 5 ml CH₂Cl₂ und 5 ml 2 n Salzsäure gelöst und 500 mg Diethylenglykoldi-*n-*butylether als interner GC-Standard hinzugegeben. Die organische Phase wurde gaschromatographisch analysiert. Die Produkte wurden durch Destillation, Kristallisation aus Methanol/Aceton-Mischungen oder säulenchromatographisch (Kieselgel, Hexan/Ethylacetat-Mischungen) isoliert.

| Tabelle 8: Reaktion von Chlorbenzol mit Acetophenon; 1 mol% PdOAc₂ | | | | | |
|---|---|---|---|---|---|
| Nr. | Ligand | Temp. | Umsatz. (%) | Ausbeute (%) | Ausbeute (%) |
| | | | | 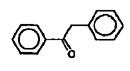 | 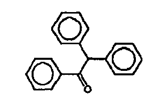 |
| | | | | Desoxybenzoin | 1,2,2-Triphenylethanon |
| 60 | 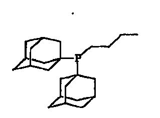 | 100 | 83 | 16 | 51 |
| | BuPAd₂ | | | | |
| 61 | 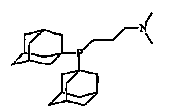 | 100 | 68 | 6 | 44 |
| | N, N-DimethylaminopropylPAd₂ | | | | |
| 62 | 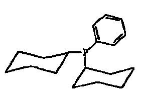 | 100 | 72 | 31 | 31 |
| | PhenylPCy₂ | | | | |
| 63 | 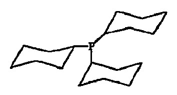 | 100 | 74 | 33 | 32 |
| | Pcy₃ | | | | |
| 64 | 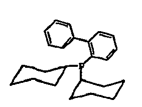 | 100 | 50 | 17 | 19 |
| | o-BiphenylPCy₂ | | | | |
| 65 | 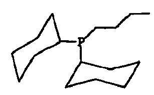 | 100 | 31 | 17 | 3 |
| | BuPCy₂ | | | | |
| 66 | 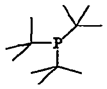 | 100 | 37 | 0 | 19 |
| | P(t-Bu)₃ | | | | |
| 67 | 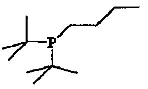 | 100 | 44 | 9 | 20 |
| | BuP(t-Bu)₂ | | | | |
| 68 | 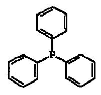 | 100 | 17 | 2 | 0 |
| | PPh₃ | | | | |

| Tabelle 9: α-Arylierung von Ketonen; 1mol% PdOAc₂; 2mol% nBuPAd₂ | | | | | | | | |
|---|---|---|---|---|---|---|---|---|
| Nr. | Aryl-X | T (°C) | Keton | Umsatz (%) | Produkt 1 . (monoaryliert) | Ausbeute^{a} (%) | Produkt 2 (bisaryliert) | Ausbeute^{a} (%) |
| 69 | 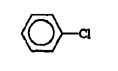 | 100 | 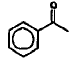 | 83 | 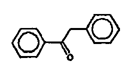 | 16 | 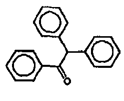 | 51 |
| | Chlorbenzol | | Acetophenon | | Desoxybenzoin | | 1,2,2-Triphenylethanon | |
| 70 | 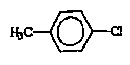 | 100 | 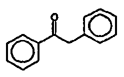 | 100 | ./. | | 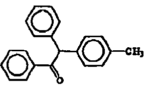 | 100 |
| | p-Chlortoluol | | Desoxybenzoin | | | | 1,2-Diphenyl-2-p-tolylethanone | |
| 71 | 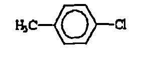 | 120 100 | 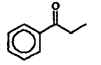 | 100 48 | 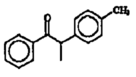 | 90 38 | ./. | |
| | p-Chlortoluol | | Propiophenon | | 1-Phenyl-2-p-tolylpropan-1-one | | | |
| 72 | 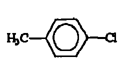 | 100 | 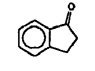 | 100 | 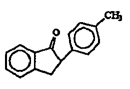 | 42 | 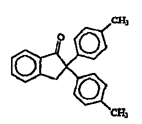 | 32 |
| | p-Chlortoluol | | 1-Indanon | | 2-p-Tolyl-1-indanon | | 2,2-Bis-(p-tolyl)-1-indanon | |
| 73 | 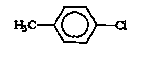 | 100 | 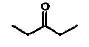 | 42 | 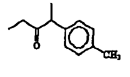 | 27 | nicht isoliert | k. A. |
| | p-Chlortoluol | | 3-Pentanon | | 2-p-Tolylpentan-3-one | | | |
| 74 | 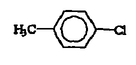 | 100 | 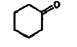 | 100 | 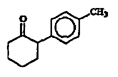 | 38 | ./. | |
| | p-Chlortoluol | | Cyclohexanon | | 2-p-Tolylcyclohexan one | | | |
| 75 | 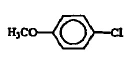 | 100 | 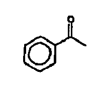 | 100 | 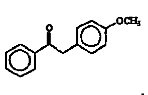 | 25 | 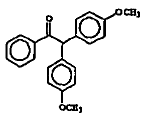 | 57 |
| | p-Chloranisol | | Acetophenon | | 2-p-Anisyl-1-phenylethanone | | 2,2-Bis-p-anisol-1-phenylethanon | |
| 76 | 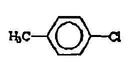 | 100 | 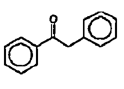 | 100 | ./. | | 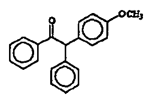 | 76 |
| | p-Chlortoluol | | Desoxybenzoin | | | | 2-p-Anisyl-1,2-diphenylethanone | |
| 77 | 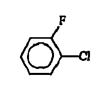 | 100 | 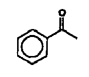 | 89 | 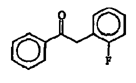 | 57 | 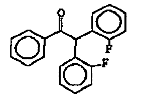 | 23 |
| | 2-Chlorfluorbenzol | | Acetophenon | | 2-(2-Fluorophenyl)-1-phenylethanon | | 2,2-Bis-(2-fluorophenyl)-1-phenylethanon | |
| 78 | 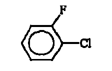 | 100 | 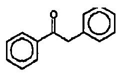 | 100 | ./. | | 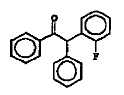 | 72 |
| | 2-Chlorfluorbenzol | | Desoxybenzoin | | | | 2-(2-Fluorophenyl)-1,2-diphenylethanone | |
| 79 | 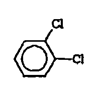 | 100 | 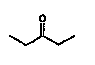 | 67 | 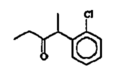 | 37 | nicht isoliert | k. A. |
| | 1,2-Dichlorbenzol | | 3-Pentanon | | 2-(2'-Chlorophenyl)-pentan-3-one | | | |

| | | | | | | | | |
|---|---|---|---|---|---|---|---|---|
| ^{a}) prozentuale Ausbeute bezogen auf in die Produkte eingebautes Arylchlorid; k. A.: nicht bestimmt. | | | | | | | | |

### Beispiel 80

### Kupplung von Arylchloriden mit Zinkorganischen Verbindungen:

Eine Suspension von 50 mmol Ethinyllithium-ethylendiamin Komplex wurde in 40 ml THF bei 0°C mit 50 mmol wasserfreiem Zinkchlorid (gelöst in 40 ml THF) versetzt. Nach Erwärmen auf RT für eine halbe Stunde wurde die Lösung erneut auf 0°C abgekühlt und mit 40 mmol 4-Chloranisol sowie 0.05 mol% Pd(OAc)₂ und 0.1 mol% Butyldiadamantylphosphin versetzt. Die Reaktionsmischung wurde bei 25 bis 50°C bis zum vollständigen Umsatz gerührt. Anschließend wurde die Lösung mit 2 M HCl-Lösung versetzt. Nach Extraktion mit Ether, waschen der Etherphase und Destillation erhält man 76% p-Methoxyphenylacetylen.

### Beispiel 81

### Kupplung mit Alkinen:

Eine Mischung von 12 mmol Trimethylsilylacetylen und 10 mmol 4-Chlornitrobenzol in 40 mL Diethylamin wird mit 0.005 mol% Pd(OAc)₂, 0.01 mol% Hexyldiadamantylphosphin und 1 mol% Cu(I)I versetzt. Die Mischung wird unter Rückfluß bis zum vollständigen Umsatz gerührt. Anschließend werden die leichtflüchtigen Bestandteile imVakuum entfernt. Der Rückstand wird in Toluol gelöst und mit Wasser gewaschen. Nach Chromatographie an Kieselgel erhält man 89% 1-(4-Nitrophenyl)-2-trimetylsilylacetylen.

### Beispiel 82

### Heck-Kupplung mit Ethylen:

50 mmol 6-Methoxy-2-bromnaphthalin, 60 mmol Kaliumcarbonat werden in 40 ml NMP gelöst und mit 0.001 mol% Pd(OAc)₂ und 0.004 mol% Butyldiadamantylphosphin versetzt. Die Mischung wird mit 20 bar Ethylendruck versetzt und bei 130 °C bis zum vollständigen Umsatz gerührt. Nach Abfiltrieren der unlöslichen Bestandteile, waschen mit alkalischer Lösung und Destillation erhält man 92 % 6-Methoxy-2-vinylnaphthalin.

### Beispiel 83

### Carbonylierungsreaktion:

20 mmol 6-Methoxy-2-bromnaphthalin, 30 mmol Triethylamin werden in 30 ml 1-Butanol gelöst und mit 0.05 mol% Pd(OAc)₂ und 0.1 mol% Butyldiadamantylphosphin versetzt. Die Mischung wird mit 3 bar CO-Druck versetzt und bei 130°C bis zum vollständigen Umsatz gerührt. Nach Abfiltrieren der unlöslichen Bestandteile, waschen mit alkalischer Lösung und Destillation erhält man 94 % 6-Methoxy-2-naphthalincarbonsäurebutylester.

## Patentansprüche

1. Phosphanliganden
Di(1-adamantyl)-i-propylphosphin,
Di(1-adamantyl)*-n-*butylphosphin,
Di(1-adamantyl)*-n-*hexylphosphin,
Di(1-adamantyl)-cyclohexylphosphin,
Di(1-adamantyl)-benzylphosphin,
Di(1-adamantyl)-pentafluorethylphosphin,
Di(3-amino-adamant-1-yl)-n-butylphosphin,
Di(3-acetyl-adamant-1-yl)-n-butylphosphin,
Di[3-(p-hydroxyphenyl)-adamant-1-yl]-methylphosphin,
Di(2-adamantyl)-i-propylphosphin,
Di(2-adamantyl)-n-butylphosphin,
Di(2-adamantyl)-t-butylphosphin,
Di(2-adamantyl)-cyclohexylphosphin,

2. Verfahren zur Herstellung der Phosphanliganden gemäß Anspruch 1
**dadurch gekennzeichnet, daß** die neuen Phosphanliganden durch Reaktion von Dihalogenadamantylphosphan bzw. Halogendiadamantyl-phosphan mit metallorganischen Reagenzien hergestellt werden.

3. Verfahren zur Herstellung der Phosphanliganden nach einem der Ansprüche 1 bis 2, **dadurch gekennzeichnet, daß** die neuen Phosphanliganden durch Umsetzung von Alkaliadamantyl- oder Alkalidiadamantylphosphiden mit organischen Elektrophilen wie Alkylhalogeniden, -pseudohalogeniden, Aldehyden oder Epoxiden hergestellt werden.

4. Verwendung der Phosphanliganden gemäß Anspruch 1 oder Phosphanliganden der Formel Ia,
(Adamantyl)₂P(Alkyl)₁ Ia
worin Adamantyl für einen in 1- oder 2-Stellung an das Phosphoratom gebundenen Adamantylrest (IIa, IIb) steht, 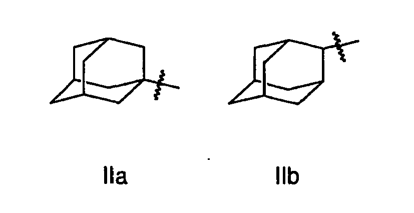 und worin Alkyl für eine C₁-bis C₁₈-Alkylgruppe
wobei sowohl die Alkylgruppe und der Adamantylrest unabhängig voneinander neben Wasserstoffatomen bis zu 10 Substituenten aufweisen können, die unabhängig voneinander C₁-bis C₈-Alkyl, O-Alkyl(C₁-C₈), OH, OCO-Alkyl(C₁-C₈), O-Phenyl, Phenyl, Aryl, Fluor, NO₂, SiAlkyl(C₁-C₈)₃, CN, COOH, CHO, SO₃H, NH₂, NH-Alkyl-(C₁-C₈), N-Alkyl(C₁-C₈)₂, P(Alkyl(C₁-C₈))₂, P(Aryl)₂, SO₂-Alkyl(C₁-C₆), SO-Alkyl(C₁-C₆), CF₃, NHCO-Alkyl(C₁-C₄), COO-Alkyl(C₁-C₈), CONH₂, CO-Alkyl(C₁-C₈), NHCHO, NHCOO-Alkyl(C₁-C₄), CO-Phenyl, COO-Phenyl, CH=CH-CO₂-Alkyl(C₁-C₈), CH=CHCOOH, PO(Phenyl)₂, PO(Alkyl(C₁-C₄))₂, PO₃H₂, PO(OAlkyl(C₁-C₆))₂, SO₃(Alkyl(C₁-C₄)) bedeuten, wobei Aryl für einen Aromaten mit 5 bis 14 Ringkohlenstoffatomen steht, wobei ein oder mehrere Ringkohlenstoffatome durch Stickstoff-, Sauerstoff- und/oder Schwefelatome ersetzt sein können, so dass ein Heteroaromat mit 1 bis 13 Ringkohlenstoffatomen vorliegt als Katalysatoren in Kombination mit Übergangsmetallkomplexen oder Übergangsmetallsalzen der VIII. Nebengruppe des Periodensystems der Elemente, wobei die Liganden in der Regel in situ zu den entsprechenden Übergangsmetallprecursorverbindungen gegeben werden beziehungsweise als Übergangsmetallphosphan-Komplexe direkt eingesetzt werden, **dadurch gekennzeichnet, dass** man die Katalysatoren zur Herstellung von arylierten Olefinen (Heck-Reaktionen), Biarylen (Suzuki-Reaktion),
und/oder Aminen aus Arylhalogeniden bzw. Vinylhalogeniden zur Carbonylierungen von Arylhalogeniden, Alkinylierungen mit Alkinen (Sonogashira-Kupplungen) und Kreuzkupplungen mit metallorganischen Reagenzien einsetzt.

5. Verwendung nach einem der vorstehenden Ansprüche, **dadurch gekennzeichnet, daß** die Liganden bei Temperaturen von 20 bis 200 °C eingesetzt werden.

6. Verwendung nach Anspruch 5, **dadurch gekennzeichnet, daß** die Temperatur auf 30 bis 180 °C, bevorzugt auf 40 bis 160 °C, gehalten wird.

7. Verwendung nach einem vorhergehenden Anspruch, **dadurch gekennzeichnet, daß** bei der katalytischen Anwendung der Phosphanligand im Überschuß zum Übergangsmetall im Verhältnis Übergangsmetall zu Ligand 1:1 bis 1:1000 eingesetzt wird.

8. Verwendung nach Anspruch 7, **dadurch gekennzeichnet, daß** das Verhältnis Übergangsmetall zu Ugand 1:1 bis 1:100 beträgt.

9. Verwendung nach einem der vorhergehenden Ansprüche, **dadurch gekennzeichnet, daß** als Übergangsmetall die Metalle Palladium, Nickel, Platin, Rhodium, Iridium, Ruthenium und Cobalt eingesetzt werden.

10. Verwendung nach Anspruch 9, **dadurch gekennzeichnet, daß** als Übergangsmetall Palladium- oder Nickelverbindungen, bevorzugt Palladiumverbindungen, eingesetzt werden.

11. Verwendung nach Anspruch 9, **dadurch gekennzeichnet, daß** für die Katalysereaktionen vorher hergestellte definierte Mono-, Di-, Tri- oder Tetraphosphankomplexe der Übergangsmetalle gemäß Anspruch 9 oder 10 eingesetzt werden.

## Claims

1. Phosphane ligands
di(1-adamantyl)-i-propylphosphine,
di(1-adamantyl)-n-butylphosphine,
di(1-adamantyl)-n-hexylphosphine,
di(1-adamantyl)cyclohexylphosphine,
di(1-adamantyl)benzylphosphine,
di(1-adamantyl)pentafluoroethylphosphine,
di(3-aminoadamant-1-yl)-n-butylphosphine,
di(3-acetyladamant-1-yl)-n-butylphosphine,
di[3-(p-hydroxyphenyl)adamant-1-yl]methylphosphine,
di(2-adamantyl)-i-propylphosphine,
di(2-adamantyl)-n-butylphosphine,
di(2-adamantyl)-t-butylphosphine,
di(2-adamantyl)cyclohexylphosphine.

2. Process for preparing the phosphane ligands according to Claim 1, **characterized in that** the novel phosphane ligands are prepared by reacting dihaloadamantylphosphane or halodiadamantylphosphane with organometallic reagents.

3. Process for preparing the phosphane ligands according to any of Claims 1 to 2, **characterized in that** the novel phosphane ligands are prepared by reacting alkali metal adamantylphosphides or alkali metal diadamantylphosphides with organic electrophiles such as alkyl halides, alkyl pseudohalides, aldehydes or epoxides.

4. Use of the phosphane ligands according to Claim 1 or phosphane ligands of the formula Ia,
(adamantyl)₂P(alkyl)₁ Ia
where adamantyl is an adamantyl radical (IIa, IIb) bound to the phosphorus atom in the 1 or 2 position, 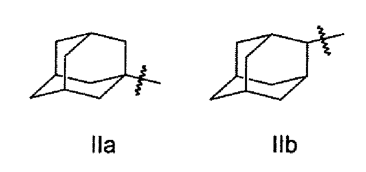 and alkyl is a C₁-C₁₈-alkyl group
where both the alkyl group and the adamantyl radical can bear, independently of one another, not only hydrogen atoms but also up to 10 substituents selected independently from among C₁-C₈-alkyl, O-alkyl(C₁-C₈), OH, OCO-alkyl(C₁-C₈), O-phenyl, phenyl, aryl, fluorine, NO₂, Sialkyl (C₁-C₈)₃, CN, COOH, CHO, SO₃H, NH₂, NH-alkyl(C₁-C₈), N-alkyl(C₁-C₈)₂, P (alkyl (C₁-C₈))₂, P(aryl)₂, SO₂-alkyl(C₁-C₆), SO-alkyl(C₁-C₆), CF₃, NHCO-alkyl(C₁-C₄), COO-alkyl(C₁-C₈), CONH₂, CO-alkyl(C₁-C₈), NHCHO, NHCOO-alkyl(C₁-C₄), CO-phenyl, COO-phenyl, CH=CH-CO₂-alkyl(C₁-C₈), CH=CHCOOH, PO(phenyl)₂, PO(alkyl-(C₁-C₄))₂, PO₃H₂, PO(Oalkyl(C₁-C₆))₂, SO₃(alkyl(C₁-C₄)), where aryl is an aromatic having from 5 to 14 ring carbons, where one or more ring carbons can be replaced by nitrogen, oxygen and/or sulfur atoms, so that a heteroaromatic having from 1 to 13 ring carbons is present, in combination with transition metal complexes or transition metal salts of transition group VIII of the Periodic Table of the Elements as catalysts, with the ligands generally being added to the appropriate transition metal precursor compounds in situ or being used directly as transition metal-phosphane complexes, **characterized in that** the catalysts are used for preparing arylated olefins (Heck reactions), biaryls (Suzuki reaction), and/or amines from aryl halides or vinyl halides for carbonylations of aryl halides, alkynylations with alkynes (Sonogashira couplings) and cross-couplings using organometallic reagents.

5. Use according to any of the preceding claims, **characterized in that** the ligands are used at temperatures of from 20 to 200°C.

6. Use according to claim 5, **characterized in that** the temperature is maintained at from 30 to 180°C, preferably at from 40 to 160°C.

7. Use according to any of the preceding claims, **characterized in that** the phosphane ligand is used in an excess over the transition metal in a ratio of transition metal to ligand of from 1:1 to 1:1000 in the catalytic application.

8. Use according to claim 7, **characterized in that** the ratio of transition metal to ligand is from 1:1 to 1:100.

9. Use according to any of the preceding claims, **characterized in that** the metals palladium, nickel, platinum, rhodium, iridium, ruthenium and cobalt are used as transition metal.

10. Use according to Claim 9, **characterized in that** palladium or nickel compounds, preferably palladium compounds, are used as transition metal.

11. Use according to Claim 9, **characterized in that** previously prepared defined monophosphane, diphosphane, triphosphane or tetraphosphane complexes of the transition metals according to Claim 9 or 10 are used for the catalytic reactions.

## Revendications

1. Ligands phosphane
di(1-adamantyl)-isopropylphosphine,
di(1-adamantyl)-n-butylphosphine,
di(1-adamantyl)-n-hexylphosphine,
di(1-adamantyl)-cyclohexylphosphine,
di(1-adamantyl)-benzylphosphine,
di(1-adamantyl)-pentafluoroéthylphosphine,
di(3-amino-adamant-1-yl)-n-butylphosphine,
di(3-acétyl-adamant-1-yl)-n-butylphosphine,
di[3-(p-hydroxyphényl)-adamant-1-yl]-méthylphosphine,
di(2-adamantyl)-isopropylphosphine,
di(2-adamantyl)-n-butylphosphine,
di(2-adamantyl)-tert-butylphosphine,
di(2-adamantyl)-cyclohexylphosphine.

2. Procédé pour la préparation des ligands phosphane selon la revendication 1, **caractérisé en ce qu'**on prépare les nouveaux ligands phosphane par réaction d'un dihalogénoadamantylphosphane ou halogéno-diadamantyl-phosphane avec des réactifs organométalliques.

3. Procédé pour la préparation des ligands phosphane selon la revendication 1 ou 2, **caractérisé en ce qu'**on prépare les nouveaux ligands phosphane par mise en réaction d'adamantyl- ou diadamantylphosphures de métaux alcalins avec des composés électrophiles organiques tels que des halogénures d'alkyle, des pseudohalogénures d'alkyle, des aldéhydes ou des époxydes.

4. Utilisation des ligands phosphane selon la revendication 1 ou de ligands phosphane de formule Ia,
(adamantyl)₂P(alkyle)₁ Ia
dans laquelle adamantyle représente un radical adamantyle (IIa, IIb) lié en position 1 ou 2 à l'atome de phosphore 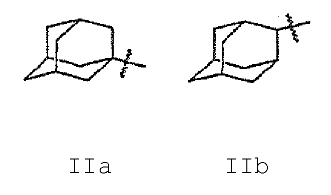 et dans laquelle alkyle représente un groupe alkyle en C₁-C₁₈
aussi bien le groupe alkyle que le radical adamantyle pouvant comporter, indépendamment l'un de l'autre, outre des atomes d'hydrogène, jusqu'à 10 substituants qui représentent, indépendamment les uns des autres, des groupes alkyle en C₁-C₈, O-alkyle(C₁-C₈), OH, OCO-alkyle(C₁-C₈), O-phényle, phényle, aryle, fluoro, NO₂, Si-alkyle (C₁-C₈)₃, CN, COOH, CHO, SO₃H, NH₂, NH-alkyle(C₁-C₈), N-alkyle(C₁-C₈)₂, P[alkyle(C₁-C₈)]₂, P(aryle)₂, SO₂-alkyle(C₁-C₆), SO-alkyle(C₁-C₆), CF₃, NHCO-alkyle(C₁-C₄), COO-alkyle(C₁-C₈), CONH₂, CO-alkyle(C₁-C₈), NHCHO, NHCOO-alkyle(C₁-C₄), CO-phényle, COO-phényle, CH=CH-CO₂-alkyle(C₁-C₈), CH=CHCOOH, PO(phényle)₂, PO [alkyle (C₁-C₄)]₂, PO₃H₂, PO[O-alkyle(C₁-C₆)]₂, SO₃[alkyle(C₁-C₄)], aryle représentant un composé aromatique ayant de 5 à 14 atomes de carbone formant le cycle, un ou plusieurs atomes de carbone formant le cycle pouvant être remplacés par des atomes d'azote, d'oxygène et/ou de soufre, de sorte qu'est présent un composé hétéroaromatique ayant de 1 à 13 atomes de carbone formant le cycle, en tant que catalyseurs en association avec des complexes de métaux de transition ou des sels de métaux de transition du groupe VIII du système périodique des éléments, les ligands étant ajoutés en règle générale in situ aux composés précurseurs de métaux de transition correspondants ou étant utilisés directement sous forme de complexes phosphane-métal de transition, **caractérisée en ce qu'**on utilise les catalyseurs pour la production d'oléfines arylées (réactions de Heck), de biaryles (réaction de Suzuki) et/ou d'amines à partir d'halogénures d'aryle ou d'halogénures de vinyle pour les carbonylations d'halogénures d'aryle, les alcynylations avec des alcynes (couplages de Sonogashira) et les couplages croisés avec des réactifs organométalliques.

5. Utilisation selon l'une quelconque des revendications précédentes, **caractérisée en ce que** les ligands sont utilisés à des températures de 20 à 200 °C.

6. Utilisation selon la revendication 5, **caractérisée en ce que** la température est maintenue à 30-180 °C, de préférence à 40-160 °C.

7. Utilisation selon une revendication précédente, **caractérisée en ce que** dans l'application catalytique on utilise le ligand phosphane en excès par rapport au métal de transition en un rapport métal de transition à ligand de 1:1 à 1:1000.

8. Utilisation selon la revendication 7, **caractérisée en ce que** le rapport métal de transition à ligand va de 1:1 à 1:100.

9. Utilisation selon l'une quelconque des revendications précédentes, **caractérisée en ce qu'**on utilise comme métal de transition les métaux palladium, nickel, platine, rhodium, iridium, ruthénium et cobalt.

10. Utilisation selon la revendication 9, **caractérisée en ce qu'**on utilise comme métal de transition des composés contenant du palladium ou du nickel, de préférence des composés contenant du palladium.

11. Utilisation selon la revendication 7, **caractérisée en ce que** pour les réactions catalytiques on utilise des complexes mono-, di-, tri- ou tétraphosphane définis des métaux de transition selon la revendication 9 ou 10, préparés précédemment.
